# EUROPEAN PATENT APPLICATION

(11) **EP 4 647 165 A1**
(43) Date of publication of application: **12.11.2025**
(21) Application number: 24738656.8
(22) Date of filing: 02.01.2024
(51) Int. Cl.: B01L 3/00

(54) **IN VITRO DIAGNOSTIC STRIP**

(30) Priority: 03.01.2023 KR 20230000859; 17.05.2023 KR 20230063498
(71) Applicant: SD Biosensor Inc., Gyeonggi-do 16690 (KR)
(72) Inventor: CHO, Young Shik, Suwon-si, Gyeonggi-do 16690 (KR); PARK, Oh Joon, Suwon-si, Gyeonggi-do 16690 (KR); KIM, Byeong Su, Suwon-si, Gyeonggi-do 16690 (KR); PARK, Hyo Lim, Suwon-si, Gyeonggi-do 16690 (KR); KIM, Dong Hun, Suwon-si, Gyeonggi-do 16690 (KR); KIM, Iek Lok, Suwon-si, Gyeonggi-do 16690 (KR)
(74) Representative: Eisenführ Speiser
(86) International application number: PCT/KR2024/000014
(87) International publication number: WO 2024/147585

(57) **Abstract**

The in vitro diagnostic strip according to an embodiment of the present invention may comprise: a strip main body into which a specimen is injected; a main deployment channel, formed on the strip main body, into which the specimen injected into the strip main body spreads; and a plurality of sub-deployment channels, branching and extending from one side of the main deployment channel, into which the specimen spreads.

## Description

### [Technical Field]

The present invention relates to an in vitro diagnostic strip, and more specifically, to an in vitro diagnostic strip for collecting a clinical specimen for in vitro diagnostics.

### [Background Art]

Recently, simple test reagents or diagnostic reagents and diagnostic devices have been variously developed to test pathogen infection caused by virus, bacteria, and the like, pregnancy, cancer, and harmful substances such as residual agricultural pesticides in specific raw materials such as food and the like.

Particularly, an in vitro diagnostic technology is a technology for quickly diagnosing a disease at the outside of a human body using materials derived from the human body, such as blood, excreta, secretion, saliva, and the like, plays an important role in determining a clinical decision, and becomes an essential and specialized factor for patient treatment.

An in vitro diagnostic device is a medical device used to test using a clinical specimen taken from a human body, such as, tissue cells, blood, urine, etc., for the purpose of diagnosis and prognosis of diseases, determination of health conditions, determination of the effectiveness of treating diseases, prevention of diseases, etc., and since the test using the in vitro diagnostic device is performed at the outside of the human body, the burden of the test on the human body is smaller than that of a test using a medical device which needs to be inserted into the human body.

In this case, an in vitro diagnostic strip, through which various test results can be extracted using one clinical specimen, needs to be developed.

### [Detailed Description of Invention]

### [Technical Problem]

One embodiment of the present invention is directed to providing an in vitro diagnostic strip from which various diagnostics are derived using one clinical specimen because a state of the clinical specimen spread through a sub-spread channel is determined to perform diagnostics.

In addition, one embodiment of the present invention is directed to providing an in vitro diagnostic strip in which a clinical specimen more smoothly flows to a sub-spread channel and a vent channel because a main spread channel, the sub-spread channel, and the vent channel are formed to have different widths and thus the channel having the relatively narrow width suctions the clinical specimen through a capillary action.

In addition, one embodiment of the present invention is directed to providing an in vitro diagnostic strip in which a label in the form of a bar code, a quick-response (QR) code, or the like is provided at one side of a strip main body, and a protocol for recognizing and automatically scanning the strip and measuring diagnostic items when the strip is inserted into an in vitro diagnostic device is implemented.

Objects to be achieved by the present invention are not limited to the above-described objects, and other objects which are not described above will be clearly understood by those skilled in the art through the following descriptions.

### [Technical Solution]

An in vitro diagnostic strip according to one embodiment of the present invention includes a strip main body to which a clinical specimen is input, a main spread channel which is formed in the strip main body and along which the clinical specimen input to the strip main body is spread, and a sub-spread channels which are branched off and extend from one side of the main spread channel as a plurality of sub-spread channels and along which the clinical specimen is spread.

The sub-spread channel vertically may extend from two sides of the main spread channel as the plurality of sub-spread channels.

A test hole through which light passes may be formed in the sub-spread channel.

A first vent hole through which outside air flows may be formed in an end portion of the main spread channel.

A width of the main spread channel may be formed to be greater than a width of the sub-spread channel.

A vent channel may be formed between the end portion of the main spread channel and the first vent hole, and a width of the vent channel may be formed to be smaller than a width of the sub-spread channel.

A second vent hole through which outside air flows may be formed in an end portion of each of the sub-spread channels.

The strip main body may include a holding part in which a clinical specimen input hole through which the clinical specimen is input is formed and a channel part coupled to one side of the holding part and having a form of a film in which the main spread channel and the sub-spread channel are formed.

The channel part may include a channel film in which the main spread channel and the sub-spread channel are formed and a first cover film and a second cover film which are attached to an upper surface and a lower surface of the channel film.

A test hole through which light passes may be formed in the sub-spread channel, and a first cover test hole and a second cover test hole which communicate with the test hole may be formed in the first cover film and the second cover film.

A first vent hole through which outside air flows may be formed in an end portion of the main spread channel, a second vent hole through which outside air flows may be formed in an end portion of the sub-spread channel, and a first cover vent hole and a second cover vent hole which communicate with the first vent hole and the second vent hole may be formed in any one of the first cover film or the second cover film.

Hydrophilic coating layers may be formed on potions of the first cover film and the second cover film corresponding to the main spread channel and the sub-spread channel.

A label to which information on a diagnostic item is input may be provided at one side of the strip main body.

### [Advantageous Effects]

According to one embodiment of the present invention, since diagnostics can be performed by determining states of a clinical specimen spread through sub-spread channels, various diagnostics can be performed using one clinical specimen.

In addition, according to one embodiment of the present invention, since a main spread channel, a sub-spread channel, and a vent channel are formed to have different widths, and the channel having the relatively narrow width suctions a clinical specimen through a capillary action, the clinical specimen can more smoothly flow to the sub-spread channel and the vent channel.

In addition, according to one embodiment of the present invention, since a label in the form of a bar code, a quick-response (QR) code, or the like is provided at one side of a strip main body, a protocol for recognizing and automatically scanning a strip and measuring diagnostic items when the strip is inserted into an in vitro diagnostic device is implemented.

### [Description of Drawings]

FIG. 1 is a perspective view illustrating an in vitro diagnostic strip according to one embodiment of the present invention.
FIG. 2 is an exploded perspective view illustrating a channel part of the in vitro diagnostic strip according to one embodiment of the present invention.
FIG. 3 is a plan view illustrating the in vitro diagnostic strip according to one embodiment of the present invention from which a first cover is removed.
FIG. 4 is a bottom view illustrating the in vitro diagnostic strip according to one embodiment of the present invention.
FIG. 5 is a perspective view illustrating an in vitro diagnostic strip according to another embodiment of the present invention.
FIG. 6 is an exploded perspective view illustrating a channel part of the in vitro diagnostic strip according to another embodiment of the present invention.

### [Modes of the Invention]

Since the present invention may be variously modified and have several embodiments, specific embodiments will be illustrated in the accompanying drawings and described in detail. However, this is not intended to limit the present invention to the specific embodiments, and it should be appreciated that all changes, equivalents, and substitutes falling within the spirit and technical scope of the present invention are encompassed in the present invention. In the description of the embodiments, certain detailed descriptions of the related art will be omitted when it is deemed that they may unnecessarily obscure the gist of the present invention.

Terms such as "first" and "second" may be used to describe various components, but the components are not limited by the above terms. These terms are used only to distinguish one component from another.

Terms used herein are only for the purpose of describing particular embodiments and are not intended to limit the present invention. Singular forms are intended to include the plural forms, unless the context clearly indicates otherwise. In the present specification, it should be understood that the terms "comprise," "comprising," "include," and/or "including" specify the presence of stated features, numbers, steps, operations, elements, components, and/or combinations thereof but do not preclude the presence or addition of one or more other features, numbers, steps, operations, elements, components, and/or combinations thereof.

In addition, throughout the specification, when components are "connected," this may not only mean that two or more components are directly connected, but may also mean that two or more components are indirectly connected through other components or are physically connected and also electrically connected, or are one component even when referred to by different names according to positions or functions thereof.

In addition, when a first component is described as being formed or disposed "on (above) or under (below)" a second component, such a description includes both a case in which the two components are formed or disposed in direct contact with each other and a case in which one or more other components are interposed between the two components. In addition, when a first component is described as being formed "on (above) or under (below)" a second component, such a description may include a case in which the first component is formed at an upper side or a lower side with respect to the second component.

Hereinafter, embodiments of an in vitro diagnostic strip will be described in detail with reference to the accompanying drawings, and when the embodiments are described with reference to the accompanying drawings, components that are the same or correspond to each other will be denoted by the same reference numerals, and redundant description thereof will be omitted.

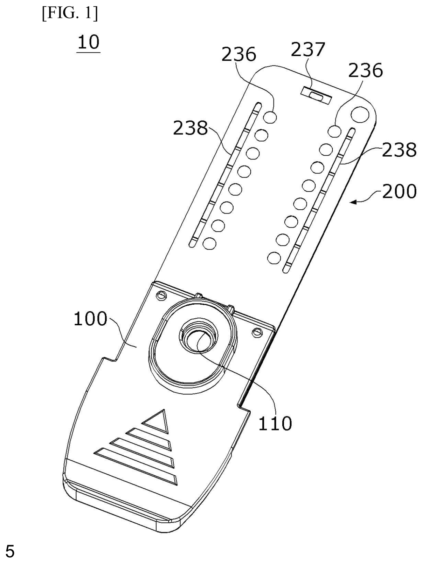

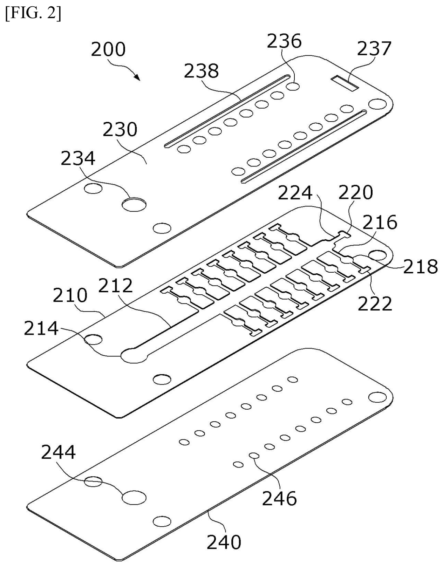

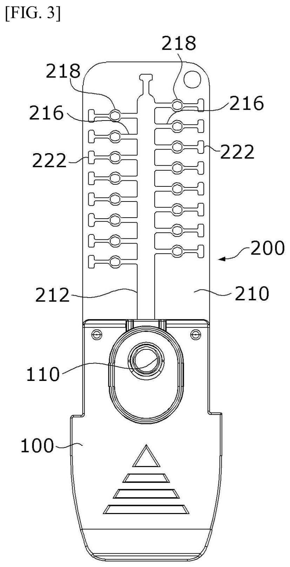

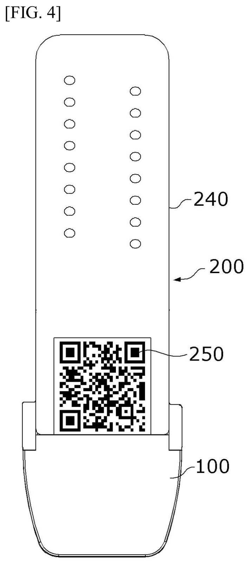

FIG. 1 is a perspective view illustrating an in vitro diagnostic strip according to one embodiment of the present invention, and FIG. 2 is an exploded perspective view illustrating a channel part of the in vitro diagnostic strip according to one embodiment of the present invention. FIG. 3 is a plan view illustrating the in vitro diagnostic strip according to one embodiment of the present invention from which a first cover is removed, and FIG. 4 is a bottom view illustrating the in vitro diagnostic strip according to one embodiment of the present invention.

According to the illustrated drawings, the in vitro diagnostic strip according to one embodiment of the present invention may include a strip main body 10, a main spread channel 212 which is formed in the strip main body 10 and along which a clinical specimen input to the strip main body 10 is spread, and a sub-spread channel 216 which is provided as a plurality of sub-spread channels 216 branched off from one side of the main spread channel 212 and along which the clinical specimen is spread.

The strip main body 10 may be a body forming an entire exterior of the in vitro diagnostic strip and may be formed in a flat plate shape. The strip main body 10 may include a holding part 100 in which a clinical specimen input hole 110, through which the clinical specimen is input, is formed and a channel part 200 coupled to the holding part 100 and having the form of a film in which the main spread channel 212 and the sub-spread channel 216 are formed.

That is, the strip main body 10 includes the holding part 100 which is held by a worker to insert the strip main body 10 into the in vitro diagnostic device and the channel part 200 coupled to one end of the holding part 100. The holding part 100 may be formed of a plastic material, and the channel part 200 may have the form of a film.

The clinical specimen input hole 110 is formed in the holding part 100, and the clinical specimen input hole 110 communicates with a channel input hole 214, a first input hole 234, and a second input hole 244 which are formed in the channel part 200. The clinical specimen input through the clinical specimen input hole 110 may be spread along the main spread channel 212 and the sub-spread channel 216 which are formed in the channel part 200.

Referring to FIG. 2, the channel part 200 may include a channel film 210 in which the main spread channel 212 and the sub-spread channel 216 are formed and cover films 230 and 240 which are attached to an upper surface and a lower surface of the channel film 210. The cover films 230 and 240 may include a first cover film 230 attached to the upper surface of the channel film 210 and a second cover film 240 attached to the lower surface of the channel film 210. The first cover film 230 and the second cover film 240 may be attached to the upper surface and the lower surface of the channel film 210 using double-sided tapes. As described above, when the channel part 200 is formed in a method of attaching the cover films 230 and 240 to the channel film 210, it may be easy to form the main spread channel 212 and the sub-spread channel 216 and may maintain a predetermined contact angle.

In this case, the main spread channel 212 and the sub-spread channel 216 may be formed to be vertically open in the channel film 210 actually constituting the channel part 200. In addition, the cover films 230 and 240 are attached to the upper surface and the lower surface of the channel film 210 to form the main spread channel 212 and the sub-spread channel 216.

The main spread channel 212 may be a channel along which the clinical specimen input to the strip main body 10 is initially spread and may be formed to extend in a longitudinal direction of the channel part 200. In addition, the plurality of sub-spread channels 216 may vertically extend from both sides of the main spread channel 212, and the clinical specimen may be spread through the plurality of sub-spread channels 216. However, the sub-spread channels 216 should not necessarily vertically extend from both sides of the main spread channel 212 and may be diagonally extend therefrom.

The plurality of sub-spread channels 216 may be formed at predetermined intervals along both sides of the main spread channel 212, and the sub-spread channels 216 disposed at one side and the other side may be alternately disposed. That is, the sub-spread channels 216 disposed at the one side may be alternately disposed between the sub-spread channels 216 disposed at the other side. When the sub-spread channels 216 at both sides are alternately disposed as described above, the clinical specimen may be easily introduced into each of the sub-spread channels 216.

Test holes 218 through which light of the in vitro diagnostic device is emitted may be formed in the sub-spread channels 216. The test holes 218 may be holes through which the light emitted from an optical module mounted in the in vitro diagnostic device passes, and states of the clinical specimen may be determined by allowing the light to pass through the test holes 218 to perform diagnostics. Since the test holes 218 are formed in the sub-spread channels 216, the worker may perform variously diagnostics from the clinical specimen detected through the test holes 218. That is, in the present embodiment, since a plurality of test holes 218 are formed, various targets as much as the number of the test holes 218 may be detected using one in vitro diagnostic device, and the test holes 218 may be used to detect the same target or different targets.

First cover test holes 236 and second cover test holes 246 may be formed in the first cover film 230 and the second cover film 240 to have openings corresponding to the plurality of test holes 218 so as to allow the light to pass through the plurality of test holes 218. The worker may easily check whether a reagent is spread to the entire channel part 200 through a process of checking from the test hole 218 formed at a location closest to a first vent hole 220.

Meanwhile, the first vent hole 220 through which outside air flows is formed in an end portion of the main spread channel 212. The first vent hole 220 may be formed in the end portion of the main spread channel 212 to allow the clinical specimen to flow in the main spread channel 212 and the sub-spread channel 216 through a capillary action. In addition, second vent holes 222 through which outside air flows are formed in ends of the sub-spread channels 216. Like the first vent hole 220, the second vent holes 222 also allow the clinical specimen to smoothly flow through a capillary action.

The first vent hole 220 and the second vent holes 222 may be formed to communicate with a first cover vent hole 237 and second cover vent holes 238 which are formed in the first cover film 230. In the present embodiment, the first cover vent holes 237 and the second cover vent holes 238 may be formed in only the first cover film 230, and the first cover vent hole 237 and the second cover vent holes 238 may be formed in the slit forms, thereby communicating with the first vent hole 220 and the second vent holes 222 disposed therein.

A vent channel 224 having a narrow flow path may be formed between the end portion of the main spread channel 212 and the first vent hole 220. In the present embodiment, the vent channel 224 is formed to have a smaller width than the main spread channel 212 to easily induce the capillary action.

More specifically, in the present embodiment, the main spread channel 212 is formed to have a greater width than the sub-spread channel 216 and the vent channel 224, and the sub-spread channel 216 is formed to have a greater width than the vent channel 224. That is, sizes of the widths of the main spread channel 212, the sub-spread channel 216, and the vent channel 224 may be larger in the order of the main spread channel 212, the sub-spread channel 216, and the vent channel 224. When the widths of the channels are different as described above, since the channel having the smaller width suctions the clinical specimen through the capillary action, the clinical specimen input to the main spread channel 212 may more smoothly flow to the sub-spread channel 216 and the vent channel 224.

According to one embodiment of the present invention, sixteen spread channels 216 may be formed in the main spread channel 212, and accordingly, sixteen test holes 218 may also be provided. According to one embodiment of the present invention, eight test holes 218 and the sub-spread channels 216 which connect the test holes 218 and the main spread channel 212 may be formed at one side of the main spread channel 212. In addition, eight test holes 218 and the sub-spread channels 216 may be provided at the other side of the main spread channel 212. However, this is only one embodiment of the present invention, the sub-spread channels 216 and the test holes 218 of which the numbers are different therefrom may be provided.

According to one embodiment of the present invention, an enzyme for performing a test may be accommodated in or applied on the first cover test hole 236 and the second cover test hole 246. Bilirubin oxidase which is one example of the enzyme may be accommodated in or applied on the first cover test hole 236 and the second cover test hole 246, but the present invention is not limited thereto. The input clinical specimen and the enzyme may react to perform the test.

Meanwhile, hydrophilic coating layers may be formed in portions of the cover films 230 and 240, in which the main spread channel 212 and the sub-spread channel 216 are formed, to implement a capillary function. In this case, the hydrophilic coating layers should not be necessarily formed in the cover films 230 and 240, the cover films 230 and 240 may be formed of a hydrophilic material, or the corresponding portions may be coated with chemical reagents. As described above, when the cover films 230 and 240 are not coated with the chemical reagents, since a function of the capillary action is degraded, and an injection speed of the clinical specimen is reduced, it may be impossible to deploy and measure the clinical specimen normally.

Referring to FIG. 4, a label 250 to which information on diagnostic items is input may be provided at one side of the strip main body 10. The label 250 may be provided in the form of a bar code, a quick-response (QR) code, or the like on a lower surface of the strip main body 10, that is, a lower surface of the channel part 200. When the label 250 is provided as described above, and the strip is inserted into the in vitro diagnostic device, a protocol for recognizing and automatically scanning the strip and measuring diagnostic items may be implemented.

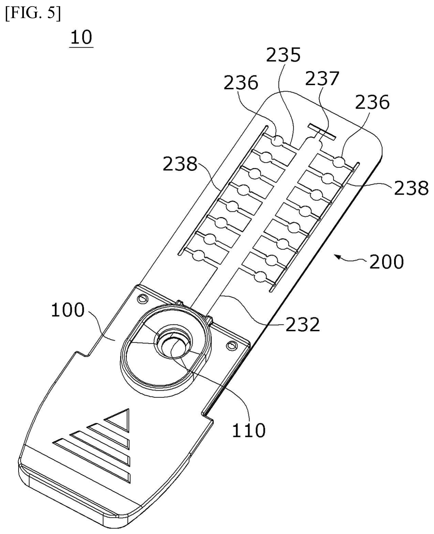

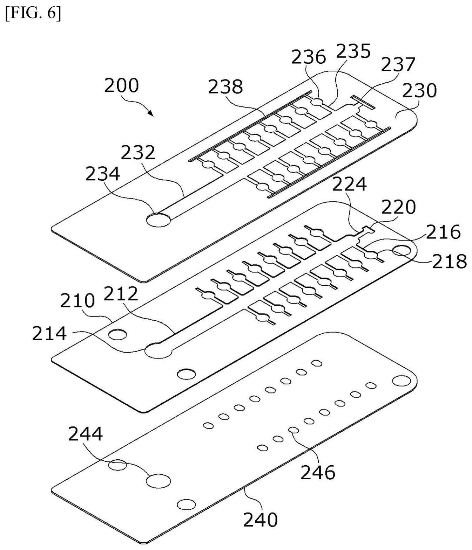

FIG. 5 is a perspective view illustrating an in vitro diagnostic strip according to another embodiment of the present invention, and FIG. 6 is an exploded perspective view illustrating a channel part of the in vitro diagnostic strip according to another embodiment of the present invention.

Referring to the drawings, in the present embodiment, components may be added to a first cover film 230 of cover films 230 and 240 constituting a channel part 200. A first input hole 234 which communicates with a channel input hole 214 is formed in the first cover film 230. A clinical specimen input to the first input hole 234 may be spread along a first main spread channel 232 and a first sub-spread channel 235. The first main spread channel 232 and the first sub-spread channel 235 may be formed to correspond to a main spread channel 212 and a sub-spread channel 216.

The first main spread channel 232 may be a channel along which the clinical specimen input to the strip main body 10 is initially spread and may be formed to extend in a longitudinal direction of the channel part 200. In addition, a plurality of sub-spread channels 235 may vertically extend from both sides of the first main spread channel 232, and the clinical specimen may be spread through the plurality of sub-spread channels 235.

While the present invention has been described above with reference to exemplary embodiments, it may be understood by those skilled in the art that various modifications and changes of the present invention may be made within a range not departing from the spirit and scope of the present invention defined by the appended claims. In addition, it should be interpreted that differences related to modifications and changes fall within the scope of the present invention defined by the appended claims.

### [Reference Numerals]

| | | | |
|---|---|---|---|
| 10: | STRIP MAIN BODY | 100: | HOLDING PART |
| 110: | CLINICAL SPECIMEN INPUT HOLE | 200: | CHANNEL PART |
| 210: | CHANNEL FILM | 212: | MAIN SPREAD CHANNEL |
| 214: | CHANNEL INPUT HOLE | 216: | SUB-SPREAD CHANNEL |
| 218: | TEST HOLE | 220: | FIRST VENT HOLE |
| 222: | SECOND VENT HOLE | 224: | VENT CHANNEL |
| 230: | FIRST COVER FILM CHANNEL | 232: | FIRST MAIN SPREAD |
| 234: | FIRST INPUT HOLE CHANNEL | 235: | FIRST SUB-SPREAD |
| 236: | FIRST COVER TEST HOLE | 237: | FIRST COVER VENT HOLE |
| 238: | SECOND COVER VENT HOLE | 240: | SECOND COVER FILM |
| 244: | SECOND INPUT HOLE | 246: | SECOND COVER TEST HOLE |
| 250: | LABEL | | |

## Claims

1. An in vitro diagnostic strip comprising:
a strip main body to which a clinical specimen is input;
a main spread channel which is formed in the strip main body and along which the clinical specimen input to the strip main body is spread; and
a sub-spread channels which are branched off and extend from one side of the main spread channel as a plurality of sub-spread channels and along which the clinical specimen is spread.

2. The in vitro diagnostic strip of claim 1, wherein the sub-spread channel vertically extends from two sides of the main spread channel as the plurality of sub-spread channels.

3. The in vitro diagnostic strip of claim 1, wherein:
the plurality of sub-spread channels extend from the two sides of the main spread channel; and
the sub-spread channels disposed at the one side of the main spread channel and the sub-spread channels disposed at the other side thereof are alternately disposed.

4. The in vitro diagnostic strip of claim 1, wherein a test hole through which light passes is formed in the sub-spread channel.

5. The in vitro diagnostic strip of claim 1, wherein a first vent hole through which outside air flows is formed in an end portion of the main spread channel.

6. The in vitro diagnostic strip of claim 1, wherein a width of the main spread channel is formed to be greater than a width of the sub-spread channel.

7. The in vitro diagnostic strip of claim 5, wherein:
a vent channel is formed between the end portion of the main spread channel and the first vent hole; and
a width of the vent channel is formed to be smaller than a width of the sub-spread channel.

8. The in vitro diagnostic strip of claim 6, wherein a second vent hole through which outside air flows is formed in an end portion of each of the sub-spread channels.

9. The in vitro diagnostic strip of claim 1, wherein the strip main body includes:
a holding part in which a clinical specimen input hole through which the clinical specimen is input is formed; and
a channel part coupled to one side of the holding part and having a form of a film in which the main spread channel and the sub-spread channel are formed.

10. The in vitro diagnostic strip of claim 9, wherein the channel part includes:
a channel film in which the main spread channel and the sub-spread channel are formed; and
a first cover film and a second cover film which are attached to an upper surface and a lower surface of the channel film.

11. The in vitro diagnostic strip of claim 10, wherein:
a test hole through which light passes is formed in the sub-spread channel; and
a first cover test hole and a second cover test hole which communicate with the test hole are formed in the first cover film and the second cover film.

12. The in vitro diagnostic strip of claim 10, wherein:
a first vent hole through which outside air flows is formed in an end portion of the main spread channel;
a second vent hole through which outside air flows is formed in an end portion of the sub-spread channel; and
a first cover vent hole and a second cover vent hole which communicate with the first vent hole and the second vent hole are formed in any one of the first cover film or the second cover film.

13. The in vitro diagnostic strip of claim 10, wherein hydrophilic coating layers are formed on potions of the first cover film and the second cover film corresponding to the main spread channel and the sub-spread channel.

14. The in vitro diagnostic strip of claim 1, wherein a label to which information on a diagnostic item is input is provided at one side of the strip main body.
